# EUROPEAN PATENT APPLICATION

(11) **EP 4 613 259 A1**
(43) Date of publication of application: **10.09.2025**
(21) Application number: 23883923.7
(22) Date of filing: 30.10.2023
(51) Int. Cl.: A61K 8/64, A61Q 19/02

(54) **PHARMACEUTICAL, DERMATOLOGICAL OR COSMETIC COMPOUNDS AND COMPOSITIONS FOR REDUCING MELANIN SYNTHESIS, STIMULATING TYPE I COLLAGEN PRODUCTION, SKIN DEPIGMENTATION AND/OR PROMOTING A SKIN ANTI-AGING EFFECT IN AN INDIVIDUAL OR ANIMAL, USES AND METHODS**

(30) Priority: 31.10.2022 BR 102022022130
(71) Applicant: Chemyunion Ltda, 18087-101 Sorocaba (BR)
(72) Inventor: KATEKAWA, Edson, Sorocaba (BR); ROGERIO PRINCIVAL, Cleverson, Sorocaba (BR); MUSSI, Lilian, Sorocaba (BR); VIDAL MAGALHÃES, Wagner, Sorocaba (BR)
(74) Representative: Hoffmann Eitle
(86) International application number: PCT/BR2023/050367
(87) International publication number: WO 2024/092332

(57) **Abstract**

The present invention describes new compounds and compositions effective for reducing melanin synthesis, stimulating the production of type I collagen, for depigmenting action, and/or anti-aging action of the skin.

## Description

### Technical Field of the Invention

The present patent application describes new compounds and compositions effective for reducing melanin synthesis, stimulating the production of type I collagen, for depigmenting action, and/or anti-aging action of the skin. The present invention is in the technical fields of pharmaceutical, dermatological and/or cosmetic treatments in humans or animals.

### Background of the Invention

Skin hyperpigmentation is considered a common dermatological manifestation and a source of discomfort for individuals affected by it, as it significantly affects psychological well-being, contributing to reduced productivity, social function and self-esteem. It is basically characterized by increased production and accumulation of melanin in the skin. The main factors responsible for this change include endocrine disorders, exposure to solar radiation, pregnancy, genetic factors, aging and skin inflammation due to acne or contact dermatitis.

Melanogenesis, the process of melanin synthesis that occurs inside melanocytes, in organelles known as melanosomes, is a complex pathway that involves a series of chemical and enzymatic reactions. Three enzymes - tyrosinase (TYR), tyrosinase-related protein 1 (TRP-1) and tyrosinase-related protein 2 (TRP-2), also known as dopachrome tautomerase (DCT) - are important mediators of this process, with TYR being exclusively necessary for the occurrence of melanogenesis (Pillaiyar T, Manickam M, Jung SH. Downregulation of melanogenesis: drug discovery and therapeutic options. Drug Discov Today. 2017 Feb;22(2):282-298). Melanogenesis begins with the oxidation of tyrosine to dopa and from this to dopaquinone by the action of TYR in the presence of molecular oxygen. This first step is limiting in the synthesis of melanin, since the rest of the reaction can occur spontaneously in an environment of physiological pH ( Pillaiyar T, Manickam M, Jung SH. Recent development of signaling pathways inhibitors of melanogenesis. Cell Signal. 2017 Dec;40:99-115). From this moment on, the presence or absence of cysteine will determine which pigment, eumelanin or pheomelanin, will be formed. In the absence of cysteine, dopaquinone is converted to leucodopachrome and then to dopachrome by auto-oxidation . Dopachrome can then, by spontaneous decarboxylation, generate 5,6-dihydroxyindole (DHI), which rapidly oxidizes and polymerizes to form insoluble, high molecular weight polymers of brown to black color, belonging to the eumelanin group. However, if TRP-2/DCT is available, dopachrome can undergo the action of this enzyme and give rise to 5,6-dihydroxyindole-2-carboxylic acid (DHICA), which, through the action of TRP-1, will also form eumelanin . In the presence of cysteine, dopaquinone reacts with this molecule, forming cysteine , which is then oxidized and polymerized, giving rise to soluble red-yellow pigments, collectively known as pheomelanins ( Pillaiyar T, Manickam M, Jung SH. Recent development of signaling pathways inhibitors of melanogenesis. Cell Signal. 2017 Dec;40:99 -115.).

Exposure to ultraviolet (UV) rays is one of the main factors stimulating melanogenesis . Exposure to these rays results in an increase in the expression of the pro- opiomelanocortin peptide (POMC) in keratinocytes. This molecule is one of the main regulatory molecules of skin pigmentation, since after its production and subsequent cleavage, different products are generated - among them, melanocyte-stimulating hormone (α-MSH) and adrenocorticotropic hormone (ACTH). These mediators are considered some of the main stimulators of melanogenesis (Pillaiyar et al., 2017b; Scherer et al., 2010). POMC is an opioid peptide precursor of several peptides such as adrenocorticotropic hormone (ACTH), β- lipotropin (β-LPH), β-endorphin (β-END) and alpha melanocyte-stimulating hormone (α-MSH), in addition to β-MSH and γ-MSH.

Melanocortin receptor (MC1-R) is predominantly expressed on the cell surface of melanocytes and has a high affinity for α-MSH. Therefore, upon binding to the MC1-R receptor, α-MSH stimulates adenylyl cyclase (AC), consequently leading to an increase in the intracellular concentration of cyclic adenosine monophosphate (cAMP). cAMP then activates protein kinase A (PKA), which translocates to the nucleus of melanocytes and phosphorylates cAMP response element-binding protein (CREB). CREB acts as a transcription factor in several genes, including melanogenesis- associated transcription factor (MITF) (Pillaiyar T, Manickam M, Jung SH. Recent development of signaling pathways inhibitors of melanogenesis. Cell Signal. 2017 Dec;40:99-115). Studies show that MITF plays an important role in melanin synthesis by stimulating the biogenesis and transport of melanosomes , as it is responsible for regulating the signaling pathways that control the differentiation and proliferation of melanocytes, in addition to stimulating the transcription of the main enzymes related to melanogenesis TYR, TRP-1 and TRP2/DCT (Gillbro JM, Olsson MJ. The melanogenesis and mechanisms of skin-lightening agents--existing and new approaches. Int J Cosmet Sci. 2011 Jun;33(3):210-21.; Pillaiyar T, Manickam M, Jung SH. Recent development of signaling pathways inhibitors of melanogenesis. Cell Signal. 2017 Dec;40:99-115 ).

There are currently several treatments for skin pigmentation disorders, but many of them are accompanied by limitations - whether related to safety, performance or pharmacotechnical issues. The main examples of lightening agents in this category include hydroquinone, arbutin and kojic acid . These agents are widely used in cosmetic or pharmaceutical applications due to their depigmenting capacity , but are not recognized for their collagen-stimulating activity. Studies suggest that topical application of hydroquinone, one of the most widely used lightening agents in the past, can disrupt the fibers of the extracellular matrix (especially collagen and elastin), leading to changes in the adrenal glands and thyroid physiology, as well as loss of skin firmness.

Furthermore, despite the initial whitening effect, hydroquinone and arbutin can cause ochronosis, a condition in which the skin becomes more pigmented than it was at the beginning of treatment. Kojic acid , an aromatic acid of fungal origin that acts as a chelating agent, has been found to promote the development of liver and thyroid cancer in rats. Contact dermatitis, rashes, burning sensations, and increased susceptibility of the skin to the effects of UV radiation are other common effects attributed to the application of these classic pharmacological substances.

The search for the state of the art revealed the following patent documents that have already aimed to present solutions to such problems.

KR1020190117311 discloses a composition containing a peptide with the sequence GlnTrpValCysSer that exhibits skin lightening activity, including melanogenesis inhibition tests. Said peptide has a structure distinct from that disclosed by the present patent application, considering both the peptide portion and any fusion molecules to the peptide.

KR1020220085368 discloses a composition containing a peptide with the sequence Caffeoyl-GlnHisGlyVal-OH that has skin lightening activity due to its anti-inflammatory activity and includes tests to reduce skin "redness". The peptide in question has a structure that is distinct from that disclosed by the present patent application, considering both the peptide portion and any fusion molecules to the peptide.

WO2016204841 discloses a composition containing a peptide with the sequence GlnLeuTrpVal that theoretically presents activity to treat and/or prevent skin hyperpigmentation (i.e. skin that is darker than desired by the individual and that is caused by melanocytes). The document presents experimental results of activity in increasing the production of type I collagen from a peptide that the inventors claim to be related to the activity of the peptide with the sequence GlnLeuTrpVal (which is never tested throughout the document). The referred peptide has a structure distinct from that disclosed by the present patent application considering both the peptide portion and possible fusion molecules to the peptide.

WO2014099609A1 discloses a composition containing a tripeptide palmitoylated composed of arginine, histidine and phenylalanine, and which has activity to reduce the appearance of melanin in the skin / modulation of melanin synthesis. The referred peptide has a structure distinct from that disclosed by the present patent application considering both the peptide portion and possible fusion molecules to the peptide.

CN102395598 / WO2010118880 presents palmitoyl-modified peptides (e.g. Pal-TyrTyrMet-NH₂) and indicates in the document that such peptides have the activity of stimulating melanin synthesis (Example 9 of the document) and the function of prolonging the effects of tanning. In other words, an effect contrary to that of the present invention. It is thus verified that such peptides can act in the stimulation / reduction of melanin synthesis, revealing that this is a complex process and that it has no specific relationship with the modification with Palmitoyl alone. The referred peptide also has a structure distinct from that disclosed by the present patent application considering both the peptide portion and possible fusion molecules to the peptide.

Thus, this patent application aims to present alternative ways of solving the problems contained in the state of the art. From what can be inferred from the literature researched, no documents were found anticipating or suggesting the teachings of the present invention.

### Summary of the Invention

Thus, the present invention aims to solve the problems in the state of the art by using new safe and effective compounds to reduce melanin synthesis. In addition, surprisingly, the new substances have a powerful stimulating effect on the production of type I collagen, an essential protein for skin firmness. Thus, in addition to its powerful depigmenting action, the present invention has an important anti-aging effect of the skin.

Thus, new compounds comprising the general formula are presented:

R1-AA1-AA2-AA3-AA4-R2,

where:
AA1 is an amino acid containing a polar side chain with a partial or formal negative charge, selected from the group Gln, Asp, Glu, Ser, Thr and His;
AA2 is an amino acid containing a polar side chain, selected from the group Gln, Asp, Glu, Ser, Thr and His;
AA3 is an amino acid containing an aromatic side chain, selected from the group Phe, Trp, Tyr, 1-naphthylalanine, 2-naphthylalanine and phenylglycine;
AA4 is an amino acid containing a nonpolar side chain, selected from the group Ala, Ile, Leu, Val Gly, Pro, Ile, Gly, norleucine, norvaline, 2-aminobutyric acid and sarcosine;
R1 is an N-terminal chain modifier selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
R2 is selected from the group consisting of -NR3R4, OR3 and -SR3, where R3 and R4 are independently selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
their stereoisomers, mixtures and/or their cosmetically or pharmaceutically acceptable salts.
In a preferred embodiment, the compound is defined by:
AA1 is selected from the Gln and Ser group;
AA2 being His;
AA3 is selected from the group Trp, Tyr;
AA4 to be Val;
where R1 is an N-terminal chain modifier selected from the N-palmitoylated or N-acetylated group and R2 is the -OH group.

In a preferred embodiment, said compound is Pal-Gln-His-Trp-Val-OH (SEQ ID No. 1), Ac-Gln-His-Trp-Val-OH (SEQ ID No. 2), Ac-Gln-His-Tyr-Val-OH (SEQ ID No. 3), Pal-Ser-His-Trp-Val-OH (SEQ ID No. 4) or Pal-Gln-His-Tyr-Val-OH (SEQ ID No. 5) wherein Pal- identifies an N- palmitoylated peptide and Ac- identifies an N-acetylated peptide.

In a preferred embodiment, said compound is for producing formulations for reducing melanin synthesis, stimulating type I collagen production, depigmenting the skin or promoting an anti-aging action of the skin in an individual or animal.

A new pharmaceutical, dermatological or cosmetic composition is also presented, comprising at least one compound as defined above.

In a preferred embodiment, the concentration of the compound is between 0.001% (w/w) and 10.00% (w/w) in the composition.

In a preferred embodiment, the composition is for formulations or for producing formulations for reducing melanin synthesis, stimulating type I collagen production, depigmenting the skin or promoting an anti-aging action of the skin in an individual or animal.

A new use of the compounds and compositions is also presented for the production of a medicine or dermatological or cosmetic composition for reducing melanin synthesis, stimulating the production of type I collagen, depigmenting the skin or promoting an anti-aging action of the skin in an individual or animal.

A new cosmetic method for reducing melanin synthesis, stimulating type I collagen production, depigmenting the skin or promoting an anti-aging action of the skin in an individual or animal is also presented, characterized by comprising the application of the composition as defined above in a cosmetically effective amount in an individual in order to reduce melanin synthesis, stimulate type I collagen production, depigment the skin or promote an anti-aging action of the skin.

A new method for reducing melanin synthesis or stimulating type I collagen production in an individual or animal is also presented, characterized by comprising the application of the composition as defined above in a dermatologically or pharmaceutically effective amount in an individual in order to reduce melanin synthesis or stimulate type I collagen production.

In one embodiment of the present invention, R1 and R2 are not proteinogenic amino acids.

Within the context of the present invention, stereoisomers of the compounds, actives and compositions described herein, mixtures and/or their cosmetically or pharmaceutically acceptable salts are also encompassed.

These and other objects of the invention may be immediately recognized by those skilled in the art and descriptions will be provided below in sufficient detail for their reproduction by a person skilled in the art.

### Brief Description of Figures

In order to better define and clarify the content of this patent application, the following figures are presented:
Figure 1: Semi-quantification of melanin pigment levels. Data obtained by recording images of histological sections of human skin fragments previously treated with a formulation without active ingredient (Placebo) and treated with the same formulation containing the peptide SEQ ID No. 1 (Pal-Gln-His-Trp-Val-OH) (at 50 ppm or 100 ppm). **p<0.01 compared to the placebo group.
Figure 2: Representative images of the effect of peptide SEQ ID No. 1 (Pal-Gln-His-Trp-Val-OH) on melanin synthesis in ex *vivo skin fragments -* Fontana Masson staining (400x magnification).
Figure 3. Semi-quantification of type I collagen levels. Data obtained by recording images of histological sections of human skin fragments previously treated with a formulation without active ingredient (Placebo) and treated with the same formulation containing the peptide SEQ ID No. 1 (Pal-Gln-His-Trp-Val-OH) (at 50 ppm or 100 ppm). **p<0.01 compared to the placebo group.
Figure 4. Representative images of the effect of peptide SEQ ID No. 1 (Pal-Gln-His-Trp-Val-OH) on the synthesis of type I collagen in *ex vivo* skin fragments - immunofluorescence (400x magnification).

### Detailed Description of the Invention

This detailed description of the invention establishes some nonlimiting definitions of the main terminologies and technical characteristics used throughout this patent application, as well as providing examples of some of the embodiments of the present invention so that it can be reproduced by a person skilled in the art.

Examples of these peptides include Pal-Gln-His-Trp-Val-OH (SEQ ID No. 1), Ac-Gln-His-Trp-Val-OH (SEQ ID No. 2), Ac-Gln-His-Tyr-Val-OH (SEQ ID No. 3), Pal-Ser-His-Trp-Val-OH (SEQ ID No. 4), and Pal-Gln-His-Tyr-Val-OH (SEQ ID No. 5), where the prefix Pal- identifies an N-palmitoylated peptide and the prefix Ac- identifies an N-acetylated peptide.

Such peptides can be obtained through various methods, preferably by chemical synthesis in solid phase or in homogeneous systems, without excluding other possibilities, such as biotechnology, hydrolysis and/or purification of proteins and other methods that may exist or may come to exist.

These peptides can be used in a concentration range between 0.001% (w/w) to 10.00% (w/w), preferably close to 0.01% (w/w), applied in a cosmetic or pharmaceutical formulation suitable for human or veterinary use.

A cosmetic or pharmaceutical formulation suitable for human or veterinary use is understood to include any and all types of solvents, dispersion media, encapsulations, permeation enhancing agents, surfactants, preservatives and other ingredients, alone or in combination, that are physiologically compatible.

It should be noted that for the description of amino acids in this document, 1 or 3 letter abbreviations may be used, as widely adopted by the scientific community and the International Nucleotide Sequence Database .

This invention further includes any and all conservative substitutions of the sequences described herein. Conservative substitution is understood as the replacement of one amino acid by another with similar hydrophobicity, polarity and/or side chain, so that there is little or no three-dimensional or functional alteration of the peptide.

### Example 1:

Example 1: Evaluation of inhibition of melanin synthesis in human skin fragments.

Skin explants (n=5), obtained from a surgical procedure performed by an ophthalmological plastic surgery clinic, were treated for 72h with a formulation containing the peptide SEQ ID No. 1 (Pal-Gln-His-Trp-Val-OH), (at 50 ppm or 100 ppm) and with the same formulation without the addition of the peptide (placebo).

After incubation with the test products as described above, the skin fragments were fixed in 4% paraformaldehyde (pH 7.4) for 24 hours. After fixation, the material was transferred to 30% sucrose solution for cryopreservation and then embedded in tissue freezing medium, followed by serial sections of approximately 10 µm thickness made by cryostat and collected directly on silanized glass slides. Then, the skin sections were stained by the Fontana Masson technique.

Subsequently, the slides were analyzed and recorded using an optical microscope coupled to a camera.

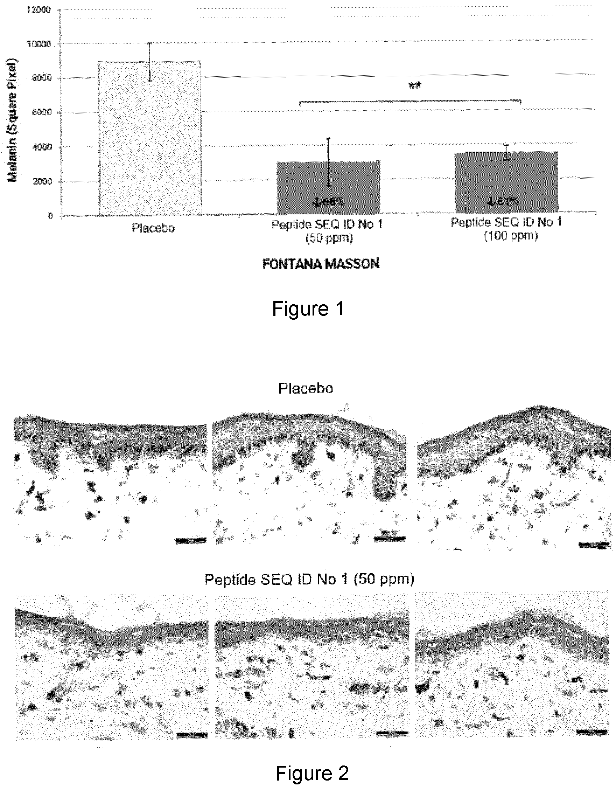

The results shown in Figures 1 and 2 demonstrate the whitening capacity of peptide SEQ ID No. 1 (Pal-Gln-His-Trp-Val-OH). At a concentration of 50 ppm, the peptide reduces melanin production by 66% compared to the placebo group. At a concentration of 100 ppm, the peptide inhibits pigment synthesis by 61% compared to the levels observed in the placebo group.

### Example 2: Evaluation of collagen synthesis stimulation in human skin fragments.

Skin explants (n=5) obtained from a surgical procedure performed by the ophthalmologic plastic surgery clinic were treated for 72 h with a formulation containing the peptide SEQ ID No. 1 ( Pal - Gln - His- Trp - Val-OH ) (at 50 ppm or 100 ppm ) and with the same formulation without the addition of the peptide (placebo). After incubation with the test products as described above, the skin fragments were fixed in 4% paraformaldehyde (pH 7.4) for 24 h. After fixation, the material was transferred to a 30% sucrose solution for cryopreservation and then embedded in tissue freezing medium, followed by serial sections of approximately 10 µm thickness made by cryostat and collected directly on silanized glass slides. The skin sections were then subjected to the specific labeling process for type I collagen for subsequent immunofluorescence analysis; the records were obtained using a fluorescence optical microscope coupled to a photographic camera.

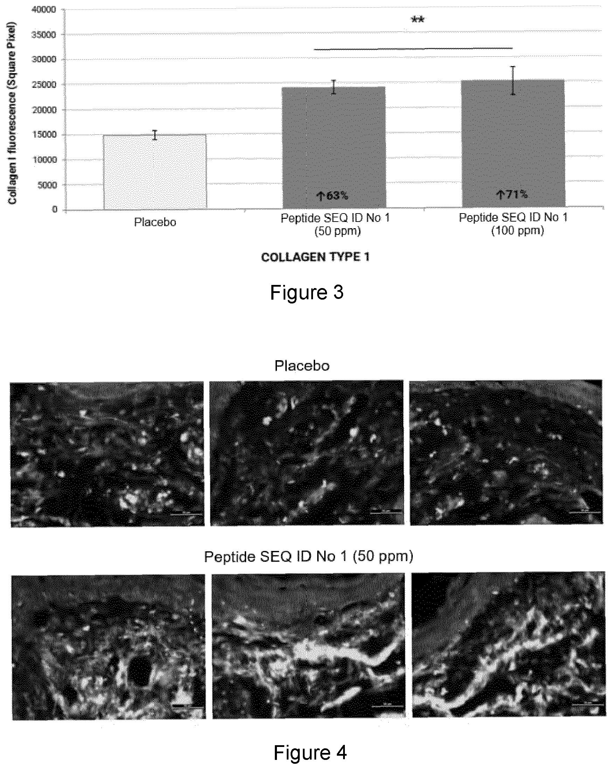

The results shown in figures 3 and 4 demonstrate the ability of peptide SEQ ID No. 1 (Pal-Gln-His-Trp-Val-OH) to stimulate the synthesis of type I collagen. At a concentration of 50 ppm, the peptide stimulated collagen I production by 63% compared to the placebo group. At a concentration of 100 ppm, the peptide promoted a 71% increase in protein synthesis compared to the levels observed in the placebo group.

### Example 3: Preparation of a cosmetic composition comprising peptide SEQ ID No. 1

A cosmetic composition was prepared whose ingredients are shown in Table 1. In a suitable container, the ingredient of Phase B was added and heated to 75-80°C. Upon reaching this temperature, the ingredient of Phase A was added under constant stirring, maintaining the temperature for 10 minutes. Then, cooling was started to 40-45°C under stirring. And then the ingredients of Phase C were added, one by one, under stirring and homogenized for another 10 minutes, obtaining a cosmetic composition with the proportions shown in Table 1. The composition is a cream suitable for topical use.

**Table 1: Composition of example 3.**

| Phase | INCI | % (w/w) | Function |
|---|---|---|---|
| A | Cetearyl Alcohol (and) Polysorbate 60 | 12.00 | Emulsifier |
| B | Water | 86.50 | Solvent |
| C | benzyl alcohol | 0.90 | Preservative |
| | Ethylhexylglycerin | 0.10 | Preservative |
| | Propanediol | 0.50 | Humectant |
| | Peptide SEQ ID No. 1 | 50 ppm | Active |

Example 4: In vivo study with the composition of Example 3, testing the efficacy of peptide SEQ ID No. 1 for depigmenting action in female volunteers.

This study was conducted over a period of 12 weeks, with measurements being taken initially at time = 0 and after 12 weeks. Twenty-one volunteers were included in the group treated with the composition of Example 3; and 20 volunteers treated with a placebo composition (same composition as in Example 3, except for the presence of the peptide). The volunteers consisted of Asian women, phototypes IV or V, between 40 and 60 years old. They applied the respective compositions to their faces. The creams were applied twice a day (morning and evening). The subjects served as their own reference and the results obtained at the different time points were compared with those obtained at the initial time zero. In addition, the results obtained with the composition containing the peptide were compared with those obtained with the placebo cream.

The effectiveness of the product is assessed by evaluating the whitening parameters (L, ITA, Melanin Index): L (Luminosity); ITA (Individual Typology) Angle ); Melanin Index. Table 2 shows the percentage variations in relation to the initial time of the parameters related to pigmentation.

**Table 2: Percentage variations in relation to the initial time of parameters related to pigmentation.**

| | VARIATION (%) | | |
|---|---|---|---|
| | Delta L (T12 weeks -T 0)/ T0 | Delta ITA (T12 weeks - T 0)/ T0 | Melanin Index (T12 weeks -T 0)/ T0 |
| Peptide Cream | +1.28% | +9.95% | -4.57% |
| Placebo Cream | +0.54% | +6.99% | -2.33% |

The examples disclosed herein are intended to merely exemplify some of the numerous forms of realization and use of the present invention, without limiting the interpretation of its scope and breadth, as well as possible alternative forms and configurational variations thereof that will be defined based on the claims presented herein.

## Claims

1. Compound, **characterized by** comprising the general formula:
R1-AA1-AA2-AA3-AA4-R2,
where:
AA1 is an amino acid containing a polar side chain with a partial or formal negative charge, selected from the group Gln, Asp, Glu, Ser, Thr and His;
AA2 is an amino acid containing a polar side chain, selected from the group Gln, Asp, Glu, Ser, Thr and His;
AA3 is an amino acid containing an aromatic side chain, selected from the group Phe, Trp, Tyr, 1-naphthylalanine, 2-naphthylalanine and phenylglycine;
AA4 is an amino acid containing a nonpolar side chain, selected from the group Ala, Ile, Leu, Val Gly, Pro, Ile, Gly, norleucine, norvaline, 2-aminobutyric acid and sarcosine;
R1 is an N-terminal chain modifier selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
R2 is selected from the group consisting of -NR3R4, OR3 and -SR3, where R3 and R4 are independently selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
their stereoisomers, mixtures and/or their cosmetically or pharmaceutically acceptable salts.

2. Compound, according to claim 1, **characterized by**:
AA1 is selected from the Gln and Ser group;
AA2 is His;
AA3 is selected from the group Trp, Tyr;
AA4 is Val;
where R1 is an N-terminal chain modifier selected from the N-palmitoylated or N-acetylated group and R2 is the -OH group.

3. Compound according to claim 1, **characterized in that** it is Pal-Gln-His-Trp-Val-OH (SEQ ID No. 1), Ac-Gln-His-Trp-Val-OH (SEQ ID No. 2), Ac-Gln-His-Tyr-Val-OH (SEQ ID No. 3), Pal -Ser-His-Trp-Val-OH (SEQ ID No. 4) or Pal-Gln-His-Tyr-Val-OH (SEQ ID No. 5);
where Pal- identifies an N- palmitoylated peptide and Ac- identifies an N-acetylated peptide.

4. Compound, according to claim 1, **characterized in that** it is for the production of formulations for reducing melanin synthesis, stimulating the production of type I collagen, depigmenting the skin or promoting an anti-aging action of the skin in an individual or animal.

5. Pharmaceutical, dermatological or cosmetic composition, **characterized by** comprising a compound as defined in any one of claims 1 to 4.

6. Composition according to claim 5, **characterized in that** the concentration of the compound is between 0.001% (w/w) and 10.00% (w/w).

7. Composition according to claim 5 or 6, **characterized in that** it is for formulations or for producing formulations for reducing melanin synthesis, stimulating type I collagen production, depigmenting the skin or promoting an anti-aging action of the skin in an individual or animal.

8. Use of a composition as defined in any one of claims 5 to 7, **characterized in that** it is for the production of a medicament or dermatological or cosmetic composition for reducing melanin synthesis, stimulating the production of type I collagen, depigmenting the skin or promoting an anti-aging action of the skin in an individual or animal.

9. Cosmetic method for reducing melanin synthesis, stimulating type I collagen production, depigmenting the skin or promoting an anti-aging action of the skin in an individual or animal, **characterized by** comprising the application of the composition as defined in any one of claims 5 to 7 in a cosmetically effective amount in an individual in order to reduce melanin synthesis, stimulate type I collagen production, depigment the skin or promote an anti-aging action of the skin.

10. Method for reducing melanin synthesis or stimulating type I collagen production in an individual or animal, **characterized by** comprising applying the composition as defined in any one of claims 5 to 7 in a dermatologically or pharmaceutically effective amount in an individual in order to reduce melanin synthesis or stimulate type I collagen production.
